# EUROPEAN PATENT APPLICATION

(11) **EP 3 721 879 A1**
(43) Date of publication of application: **14.10.2020**
(21) Application number: 19425026.2
(22) Date of filing: 08.04.2019
(51) Int. Cl.: A61K 31/19, A61P 17/02, A23K 10/00

(54) **EXTERNAL USE OF METHIONINE HYDROXYANALOGUE AND ITS DERIVATIVES, ALONE OR IN COMBINATION WITH OTHER ELEMENTS, FOR THE PREVENTION AND CONTROL OF PIG AGGRESSIVENESS**

(71) Applicant: Duregger S.r.l., 12038 Savigliano (CN) (IT)
(72) Inventor: Baricco, Giuseppe, I-10145 Torino (IT)
(74) Representative: Garavelli, Paolo

(57) **Abstract**

A use of methionine hydroxyanalogue or its derivatives is described, administered externally on the skin of pigs in order to prevent, control or treat episodes of aggression among animals that are observed in various situations of modern breeding.

## Description

The present invention relates to the use of mixtures containing methionine hydroxyanalogue or its derivatives administered externally on the skin of pigs in order to prevent or control the effects of the aggressiveness of pigs on farm.

Methionine and its precursors (methionine hydroxyanalogue, CAS 583-91-5, Formula C5H1003S) have a wide and consolidated use in animal nutrition, methionine being the second limiting amino acid in the diets of bred animals, after lysine. Therefore, these products have been used for years in animal nutrition. In particular, in the EC they are included in Annex 1 of EC Regulation 1831/2003, which regulates the placing on the market of additives for zootechnical use. Methionine hydroxyanalogue is a brown liquid with a characteristic sulfur odor. The pH of the product is less than 1, and is higher in density than water: however it is perfectly miscible with all the water-soluble substances, obviously except for specific chemical compatibility.

Per os and inhalation, methionine hydroxyanalogue does not pose significant toxicity problems, while there are reported cases of irritation due to skin contact, and more frequent and intense reactions in case of contact with the cornea.

The hydroxyanalogue methionine tends to pass through porous materials, such as skin, and to remain there for a certain time, which is among the rational bases of the present invention.

Recent normative indications on the protection of animal welfare on farms (Directive 2008/120 / CE and Recommendation (EU) 2016/336) place particular attention to the elimination of tail cutting as a routine operation in productive farms. The progressive increasing interaction between the instances of the so-called "civil society" (almost always urbanized) and the productive methodologies adopted in intensive farms (almost always emotionally unacceptable for urban populations) has created the conditions because organized pressure groups, motivated by legitimate aspirations for animal welfare, obtained the result of making tighter and closer constraints to the intensive exploitation of animals for productive purposes.

The cutting of pigs' tails at a young age (within the first 7 days of life) has always been practiced by farmers when, in the later stages of life, pigs are required to coexist in relatively narrow spaces. In fact the movement of the whole tail acts as an excitation factor for the animals which, intrigued by the movement, explore it and nibble it. Not infrequently these exploration activities result in authentic bites, with the creation of wounds that on the one hand can become infected, paving the way for systemic infections, on the other hand, offering the cohabiting animals the taste of blood, excite an even more aggressive behavior.

Obviously, this does not happen if animals have large open spaces available, but this is not the standard condition of modern pig production.

Similarly, in more distant times, Directive 2001/93 EC has banned the breeding of sows in single cages, excluding the period of birth and lactation and the one relating to the subsequent insemination up to the confirmation of pregnancy. This generates the situation where approximately 2-3 times a year the sows are grouped, and each time they must establish the hierarchical order within the newly formed group. Pigs establish social priority through combat, and consequently in this case too, episodes of struggle are commonly observed on farms, which can result in wounds to the tail and vulva, and in extensive abrasions on the flanks of animals.

Routine recourse to systemic sedatives is expressly prohibited by Directive 2001/93 EC, and adaptation of breeding facilities is not always possible, therefore there is a need for a composition that fully meets the following requirements:
- provide an efficient means to inhibit, or control, the exaggerated phenomena of social interaction between bred pigs
- do not be a sedative, in accordance with the provisions on animal welfare
- be safe for animals and the environment
- be in accordance with the current and foreseeable rules regarding the management criteria of pig farms.

The above elements, others, and the advantages of the invention, as will appear from the following description, are obtained with the use of methionine hydroxyanalogue in combination or not with other elements as claimed in Claims 1 and 6. Preferred forms and non-trivial variants of the present invention are claimed in the dependent Claims.

It is understood that all enclosed Claims are an integral part of the present description.

The present invention will be better described by some preferred forms thereof, provided as a nonlimiting example, with reference to the accompanying drawings, in which:
- Figure 1 is a graphic representation of the result of the external use of methionine hydroxyanalogue in combination with other elements on the struggles between sows at the time of the creation of groups;
- Figure 2 is a graphical representation of the result of the external use of methionine hydroxyanalogue in combination with other elements on the struggles between pigs of about 20 kg at the time of the creation of groups for fattening; and
- Figure 3 is a graphical representation of the result of the external use of methionine hydroxyanalogue in combination with other elements on the weaning of piglets with untouched tail.

It will be immediately obvious that numerous variations and modifications (for example referred to the shape, dimensions, combinations and parts with equivalent functionality) can be carried out as described, without failing to the purpose of the invention as it appears from the enclosed Claims. Methionine hydroxyanalogue has a characteristic odor, unpleasant both for humans and for pigs, and is characterized by almost no direct or indirect toxicity, both on animals and on the environment.

The characteristic of the substance to easily penetrate through the stratum corneum of the skin favors the persistence of the bad smell on the skin for a certain time after administration, even when it occurs by simple nebulization on the area to be treated.

The particularly acidic pH of methionine hydroxyanalogue does not recommend its use in purity, in order to avoid possible eye irritation due to small amounts that can come into contact with the cornea of the animals.

The discovery consists in having understood that the methionine hydroxyanalogue distributed on the parts exposed to attack or to simple exploratory curiosity of the animals inhibits in a sensitive and lasting way the intensity of this activity, because of the smell (foreign, unpleasant and persistent) that discourages the phenomena of aggression and exploration of one animal towards the other.

So, the merits of the invention are two:
1. providing a new, safe, and reasonably effective tool to combat widespread aggressive behaviors of pigs raised, with a new use of a known and legally usable substance
2. reducing the indiscriminate use of molecules sedatives for the prevention or treatment of these behaviors.

The invention is now described in detail.

The treatment is topical, as no pharmacological type activity is foreseen: instead it uses sensory elements.

The pig is endowed with a particularly developed sense of smell (it is probably the animal with the most developed sense of smell), and is endowed with an equally sensitive sense of taste: it is not instead endowed with a view neither particularly acute nor sensitive.

Therefore, to inhibit the behavior of social interaction through the mouth and the nose, it is necessary to mask the object of his curiosity (in this case the tail, the ears, the vulva and the hips of the like) with substances that:
1. by olfactory route discourage any approach
2. by gustatory route (where interaction by the mouth is still present), they inhibit its continuation.

The rationale of invention is based on these concepts.

The methionine hydroxyanalogue sprayed on the skin emits an unpleasant smell that tends to keep the pigs away from each other.

The persistence of this unpleasant smell can be increased by using propylene glycol, which is widely used in cosmetics to promote the penetration of active ingredients through the skin. Also propylene glycol is an ingredient widely used in animal nutrition, present in EC Regulation 1017/17 at No. 13.11.1. It is a slightly sweet tasting substance. From the chemical point of view it is a diol, an aliphatic organic compound with two alcoholic functions. It comes in the form of a colorless viscous liquid, highly hydrophilic, soluble in water, miscible with water, alcohol and acetone. Propylene glycol has wetting capacity, a good solvent action for aromas and fragrances and is considered an important vehicle for active substances. It has a mild moisturizing action. Is among the most used humectants in the dermocosmetic field to favor the hydration and penetration of the active ingredients through the dermis: therefore it is evident that a substance (methionine hydroxyanalogue) that already in itself has a good ability to penetrate through the skin, where dispersed in propylene glycol, this specific characteristic will be increased.

However some pigs, overcoming the repellence induced by the smell, can anyway approach to their similar: to discourage the continuation of this attitude after the first approach, in the preparation can be added substances that act directly, and still in an unpleasant way, on the mouth of animals when they come into contact with it. For this purpose it is possible to use the extract of *castanea sativa* (flavoring feed additive present in Annex 1 of Reg. 1831/2003, category 2.b) which, being rich in tannins, induces in the tongue of those who come into contact with it the phenomenon of astringency, a sensation of dryness and roughness (set the mouth on edge) perceived in the oral cavity. The sensation is perceived not only on the tongue, but also in the palate and gums; moreover it is received by the brain in a different way, more acute, than the flavors.

The typical composition of the preparation is the following:
1. Methionine hydroxyanalogue 25%
2. Liquid castanea sativa extract 25%
3. Propylene glycol 45%
4. Excipients 5%

The product has no shelf life problems and appears as a viscous brown liquid, of medium density, with a pungent and characteristic odor.

It can also be used on open wounds (wounds, scratches) on which the acid pH of the product and the presence of the tannins contained in the extract of *castanea sativa* act as a coagulation and disinfection factor, favoring healing and subsequent re-epithelialization, with the formation of a blackish crust of characteristic odor that protects the wound and discourages further exploration by other animals.

### DESCRIPTION OF THE TREATMENT

The product must be sprayed on the parts of animals subjected to exploration or aggression by the similar ones.

The treatment is different depending on the breeding condition that is intended to be controlled:

### A) CREATION OF SOWS GROUPS

In this case it is advisable to abundantly spray the body of the animals the evening before the event, while they are still contained in a single cage. The parts to be sprayed are the snout, the ears, the hips, and the perineal area including the tail. The average dose necessary for this operation is 100/150 gr. of product per sow.

During the night the product is dried and penetrates into the skin layer. In the case of particularly aggressive animals, further nebulization is possible at the time of mixing.

A second spraying is recommended in the evening after mixing, while in the following days it will be sufficient to selectively spray those animals that are still subject to particular aggression by others.

### B) CREATION OF PIGLET GROUPS

In this case the treatment foresees the prompt spraying of the sensitive areas (mainly the auricles and the perineal area, including the tail) of the piglets at the time of group formation. The average dose needed to spray a piglet is 30-50 gr. of product. A second spraying at a distance of 12 hours is recommended, while subsequently it will be sufficient to selectively spray those animals that are still subject to particular aggression on the part of others.

### C) WEANING OF PIGLETS WITH UNCUTTED TAIL

In this case the treatment involves the prompt spraying of the sensitive areas (mainly the auricles and the perineal area, including the tail) of piglets at the time of weaning. The average dose needed to spray a piglet is 20-40 gr. of product. A second spraying at a distance of 12 hours is recommended, and for the 3-4 days following a spraying per day. Subsequently it will be sufficient to selectively spray those animals that are still the object of particular aggression on the part of others, in particular on the tail.

### D) TREATMENT OF SWINE CANNIBALISM

Sometimes in fattening sheds acute episodes of aggression occur among pigs, which can recognize various triggering factors (overcrowding, poor air quality, environmental dustiness, excessive light, fungal contamination of feed, genetic predisposition). These acute episodes not infrequently result in authentic episodes of cannibalism, due to the animals' fury on open wounds.

In these cases a continuous spraying (even 2-3 times a day) with the product on the open lesions and on the neighboring areas, taking care to provide a final spraying in the evening (when normally the episodes of aggression tend to decrease), from one side allows the control of possible infections, on the other it discourages attacking pigs in continuing this activity. Treatment should be continued until a dark and solid crust appears on the lesions.

The invention will now be described through nonlimiting examples.

### RESULTS OF THE PRELIMINARY TRIALS

Scientifically organized tests, under the control of University or official institutes, have not been conducted, however through private and confidential relationships numerous cases have been treated in order to obtain preliminary information on the efficacy of the treatment of the invention in the different conditions of use .

The tested product had the following composition:

| | |
|---|---|
| Methionine hydroxyanalogue | 25% |
| Liquid extract of *cassanea sativa* | 25% |
| Propylen glycol | 45% |
| Water and excipients | 5% |

This product has been provided to:
Ten breeding companies to test the effect on the formation of groups of sows
Five fattening units to test the effect at the time of the formation of fattening groups on piglets weighing 20-30 kg.
Five breeding companies to test the effect on weaning of whole-tailed piglets.

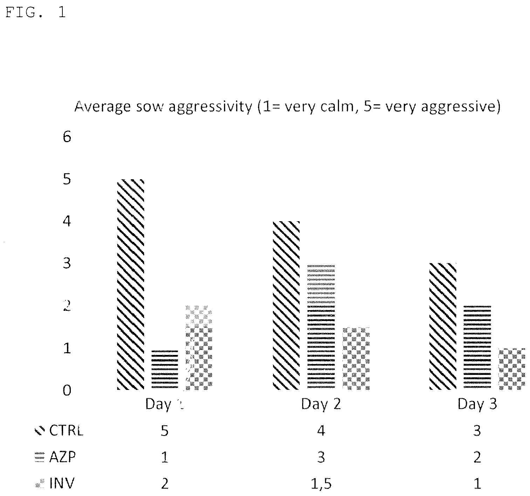

The results and data collection methods are now illustrated in detail:
In the first experiment (Figure 1.) ten farmers were asked to respect the treatment protocol illustrated, and to express their judgment on the aggressiveness of the sows with respect to three conditions:
1) Sows not subjected to any treatment (CTRL)
2) Sows subjected to injection with Stresnil (Azaperone) 1.5 ml / head at the time of mixing (AZP)
3) Sows subjected to the treatment with the product and the protocol object of the invention (INV)

The degree of judgment varied from 1 to 5, where 1 corresponded to sows not at all aggressive, 5 to extremely aggressive sows.

As is quite obvious, the sows quenched by injection on the first day showed a quieter behavior, but already starting from the second day, once the effect of the drug was exhausted, their normal fighting attitude almost resumed.

Sows neither sedated nor treated have shown normal aggressive behavior.

Sows subjected to invention have had a profile of normal behavior (aggression present, and decreasing with time), but of decidedly lower intensity, and compatible with the parameters of animal welfare. In the following days, the persistence of the bad smell on their skin discouraged social interactions more than in other groups.

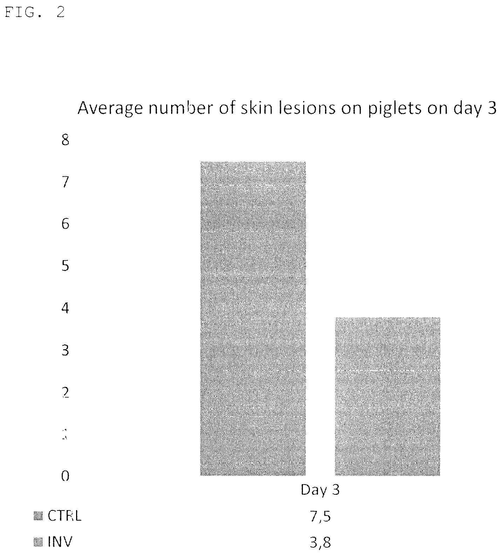

In the second experiment (Figure 2.) five breeders were asked to apply the recommended protocol when piglets were put into stalls for fattening, and to compare it with homogeneous, contemporary and untreated groups. On day 3 the stables were visited, and the number of visible lesions (wounds, abrasions) on the piglets was detected, expressed with the average number of skin lesions / piglets.

The treatment resulted in a marked reduction of visible lesions, a clear sign of less aggressiveness among the animals in the previous days.

In this condition the efficacy of the treatment was proportionally less evident compared to experiment 1 on sows, although it obtained a non-marginal result (- 50% of skin lesions).

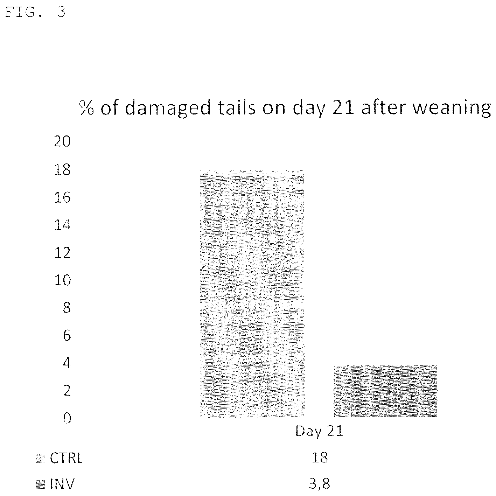

In the third experiment (Figure 3.) five breeders were asked to apply the suggested protocol based on weaned piglets with the uncut tail, comparing them with similar untreated groups.

The result has been checked by verifying on the 21st day after weaning the number of injured tails (which were therefore subject to social interaction), expressed as a percentage.

The treatment has discouraged social interactions among piglets, in particular on the uncutted tails, preventing possible ascending infections and favoring the disinterest of the animals towards the factor of excitement and curiosity represented by the whole tail.

## Claims

1. Use of methionine hydroxyanalogue or its derivatives applied to the skin of pigs by spraying or misting or in any other way in order to control, prevent, or cure the effects of swine aggression, as manifested in various conditions of modern breeding.

2. Use according to Claim 1, in which the dosage of methionine hydroxyanalogue in preparations is from 10 to 90%, preferably from 20 to 30%.

3. Use according to Claim 1, in which the preparation of the product is in liquid form, in any form that can be used to be sprayed onto the skin of animals.

4. Use according to Claim 3, in which the finished product is marketed as a feed, as a feed additive, as a medical device, a biocide, a drug or a cosmetic.

5. Use according to Claim 1, in which the package or nebulization tool used for the administration of the finished product in accordance with Claim 3 and 4 is useful for increasing the effectiveness of the product.

6. Composition, in which the basic active ingredient, namely methionine hydroxyanalogue or a derivative thereof, is associated with any other substance compatible with the product category of the product, for example glycerin, propylene glycol, organic acids, fatty acids, plant extracts, vitamins or aromas designed to enhance the effectiveness of the product on the skin of animals.
